(19) Europäisches Patentamt
European Patent Office
Office européen des brevets

(11) Veröffentlichungsnummer : **0 171 016**
**B1**

(12) # EUROPÄISCHE PATENTSCHRIFT

(45) Veröffentlichungstag der Patentschrift : **09.11.88**

(51) Int. Cl.⁴ : **C 07 H 13/12**

(21) Anmeldenummer : **85109529.9**

(22) Anmeldetag : **29.07.85**

(54) **In alpha-Stellung durch eine Isocyaniddichlorid-Gruppe substituierte cyclische Ether und Verfahren zu ihrer Herstellung.**

(30) Priorität : **10.08.84 DE 3429432**

(43) Veröffentlichungstag der Anmeldung : **12.02.86 Patentblatt 86/07**

(45) Bekanntmachung des Hinweises auf die Patenterteilung : **09.11.88 Patentblatt 88/45**

(84) Benannte Vertragsstaaten : **BE CH DE FR GB IT LI NL**

(56) Entgegenhaltungen :
**EP-A- 0 142 094**
**SYNTHESIS, Nr. 6, Juni 1984, Seiten 509,510, Georg Thieme Verlag, Stuttgart, DE; M.J. CAMARASA et al.: "A new procedure for the synthesis of glycosyl isothiocyanates"**
**CHEMICAL ABSTRACTS, Band 96, Nr. 17, 26. April 1982, Seite 796, Zusammenfassung Nr. 143202k, Columbus, Ohio, US; OGURA HARUO et al.: "Studies on nucleoside analogs. XXIV. Mono- and disaccaride isothiocyanates", & HETEROCYCLES 1982, 17(Spec. Issue) 87-90**

(73) Patentinhaber : **BAYER AG**
**Konzernverwaltung RP Patentabteilung**
**D-5090 Leverkusen 1 Bayerwerk (DE)**

(72) Erfinder : **Hassel, Tillmann, Dr.**
**Morgengraben 14**
**D-5000 Köln 80 (DE)**
Erfinder : **Müller, Hanns Peter, Dr.**
**Im Kerberich 6**
**D-5068 Odenthal-Blecher (DE)**
Erfinder : **Böshagen, Horst, Dr.**
**Wiesenstrasse 4**
**D-5657 Haan (DE)**

**EP 0 171 016 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue in α-Stellung durch eine Isocyaniddichlorid-Gruppe substituierte cyclische Ether und ein Verfahren zur ihrer Herstellung.

Es wurden neue in α-Stellung durch eine Isocyaniddichlorid-Gruppe substituierte cyclische Ether der Formel

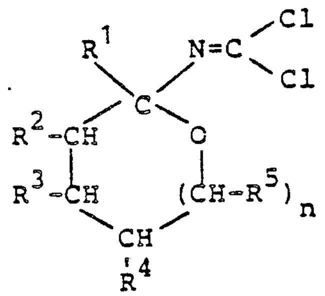

(I)

gefunden, in der

$R^1$ eine Acyloxymethylgruppe oder — vorzugsweise — Wasserstoff bedeutet,

n 0 oder 1 ist und

$R^2$, $R^3$, $R^4$ und $R^5$ unabhängig voneinander für Wasserstoff, Halogen, eine Nitro-, Acyloxy- oder Acyloxymethyl-Gruppe oder den Rest eines cyclischen Ethers der Formel

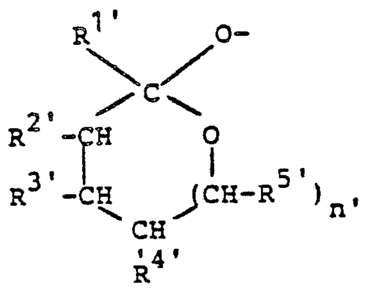

(II)

stehen, in der n', $R^{1'}$, $R^{2'}$, $R^{3'}$, $R^{4'}$ und $R^{5'}$, unabhängig voneinander und unabhängig von den ihnen entsprechenden Werten n, $R^1$, $R^2$, $R^3$, $R^4$ und $R^5$ in Formel I, die für n, $R^1$, $R^2$, $R^3$, $R^4$ und $R^5$ unter Formel I angegebene Bedeutung haben, mit der Maßgabe, daß mindestens einer der Reste $R^1$, $R^2$, $R^3$, $R^4$ und $R^5$ und mindestens einer der Reste $R^{1'}$, $R^{2'}$, $R^{3'}$, $R^{4'}$ und $R^{5'}$ eine Acyloxymethyl- oder Acyloxy-Gruppe ist — vorzugsweise sind mindestens zwei der Reste $R^2$, $R^3$, $R^4$ und $R^5$ und mindestens zwei der Reste $R^{2'}$, $R^{3'}$, $R^{4'}$ und $R^{5'}$ eine Acyloxymethyl- oder Acyloxy-Gruppe — und daß höchstens einer der Reste $R^{2'}$, $R^{3'}$, $R^{4'}$, oder $R^{5'}$ für den Rest eines cyclischen Ethers der Formel II steht.

Bei den bevorzugten cyclischen Ethern der Formel I handelt es sich um acylierte (Desoxy-)Zucker mit glykosidisch gebundener Isocyaniddichlorid-Gruppe. Je nach dem, ob einer der Reste $R^2$, $R^3$, $R^4$ oder $R^5$ für den Rest eines cyclischen Ethers der Formel II steht und ob in dieser Formel II wiederum einer der Reste $R^{2'}$, $R^{3'}$, $R^{4'}$ oder $R^{5'}$ der Rest eines cyclischen Ethers der Formel II ist, können diese acylierten Zucker Mono-, Di- oder Oligosaccharide sein. Besonders bevorzugt sind Mono- und Disaccharide, d. h. die cyclischen Ether der Formel I, die den Rest des cyclischen Ethers der Formel II nicht oder nur einmal aufweisen.

Besonders bevorzugt sind solche erfindungsgemäßen cyclischen Ether der Formel I, wenn in dieser

$R^1$ Wasserstoff und

n 0 oder 1 ist und

$R^2$, $R^3$, $R^4$ und $R^5$ für eine Acyloxy- oder Acyloxymethylgruppe stehen, oder einer der Reste $R^2$, $R^3$, $R^4$ oder $R^5$ der Rest eines cyclischen Ethers der Formel II ist, und die verbleibenden $R^2$, $R^3$, $R^4$, $R^5$ für eine Acyloxy- oder Acyloxymethyl-Gruppe stehen, wobei in Formel II

$R^{1'}$ für Wasserstoff oder eine Acyloxymethyl-Gruppe,

n' für 0 oder 1 und

$R^{2'}$, $R^{3'}$, $R^{4'}$ und $R^{5'}$ für eine Acyloxy- oder Acyloxymethyl-Gruppe stehen.

Vorzugsweise leiten sich die Acylreste in den Acyloxy- und Acyloxymethyl-Gruppen von der gleichen Säure ab.

Für die Reste $R^2$, $R^{2'}$, $R^3$, $R^{3'}$, $R^4$, $R^{4'}$, $R^5$ und $R^{5'}$ kommt als Halogen vorzugsweise Fluor, Chlor oder Brom in Betracht.

Die Acylreste leiten sich vorzugsweise von niederen aliphatischen, z. B. $C_1$-$C_8$-Alkancarbon- oder -sulfonsäuren, wie der Essigsäure, Propionsäure, Buttersäure, der Methansulfonsäure und der Ethansulfonsäure, oder aromatischen Carbon- und Sulfonsäuren, wie der Benzoesäure und der p-Toluolsulfonsäure ab.

Bevorzugte Vertreter der Acylreste sind die in der Zuckerchemie vielfach verwendeten Acylreste, wie der Methansulfonyl(Mesyl)-Rest, der p-Toluolsulfonsäure(Tosyl)-Rest und vor allem der als Schutzgruppe bekannte Acetyl- und der Benzoylrest.

Als Vertreter der bevorzugten erfindungsgemäßen cyclischen Ether der Formel I seien beispielsweise die folgenden acylierten Zucker mit glykosidisch gebundener Isocyaniddichlorid-Gruppe genannt :

2,3,4,6-Tetraacetyl-D-glucopyranosyl-1-isocyaniddichlorid ;
2,3,4-Tribenzol-D-ribopyranosyl-1-isocyaniddichlorid ;
2,3,5-Tribenzoyl-D-ribofuranosyl-1-isocyaniddichlorid ;
2,2',3,3',4',6,6'-Heptaacetyl-D-lactosyl-1-isocyaniddichlorid ;
2,2',3,3',4',6,6'-Heptaacetyl-D-cellobiosyl-1-isocyaniddichlorid ;
3-Desoxy-3-chloro-2,4,6-triacetyl-D-glucosyl-1-isocyaniddichlorid ;
2-Desoxy-2-p-toluolsulfonyloxy-3,4,6-triacetyl-D-glucosyl-1-isocyaniddichlorid, aber auch 2,3,4-Triacetyl-6-desoxy-6-nitroglucopyranosyl-1-isocyaniddichlorid und 2-Desoxy-3,4,6-tribenzoyl-D-glucopyranosyl-1-isocyaniddichlorid, sowie 2,3,4-Triacetyl-L-arabinopyranosyl-isocyaniddichlorid und 2,3,4-Triacetyl-D-xylopyranosyl-isocyaniddichlorid.

Die Erfindung betrifft ferner ein Verfahren zur Herstellung der in α-Stellung durch eine Isocyaniddichlorid-Gruppe substituierten cyclischen Ether der Formel I ; das Verfahren ist dadurch gekennzeichnet, daß man in α-Stellung durch eine Isothiocyanat-Gruppe substituierte cyclische Ether der Formel

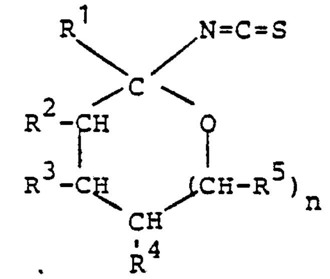

(III)

in der n, $R^1$, $R^2$, $R^3$, $R^4$ und $R^5$ die unter Formel I angegebene Bedeutung haben, mit mindestens 2 Mol Chlor, vorzugsweise 2 bis 6 Mol, besonders bevorzugt 2 bis 3 Mol Chlor, je Mol Isothiocyanat-Gruppe in einem inerten Lösungsmittel bei Temperaturen von —40° bis + 80 °C, vorzugsweise von — 20 bis + 60 °C, besonders bevorzugt von 0 bis + 25 °C umsetzt.

Überraschenderweise erhält man die in α-Stellung durch eine Isocyaniddichlorid-Gruppe substituierten cyclischen Ether auch dann in hohen Ausbeuten und großer Reinheit, wenn man mit einem Überschuß an Chlor arbeitet.

Verfahren zur Herstellung von Isocyaniddichloriden aus Isothiocyanaten durch Umsetzung mit Chlor sind bekannt (s. Houben-Weyl, Methoden der Organischen Chemie, 4. Auflage, Band E 4, Seiten 522 ff.). Es war jedoch überraschend daß diese bekannte Umsetzung auch mit Acyloxygruppen aufweisenden Ethern mit in α-Stellung befindlicher Isothiocyanat-Gruppe gelingt, da aus Weissermel/Arpe : Industrielle Organische Chemie, 2, Auflage, Seite 172 ; und Houben-Weyl, Methoden der Organischen Chemie, 4. Auflage, Band V/3, Seiten 623 ff, zu erwarten war, daß bevorzugt als Konkurrenzreaktion die Chlorierung der Acyloxygruppe eintritt.

Das erfindungsgemäße Verfahren sei für die Tetraacetylglukose mit glykosidisch gebundener Isothiocyanat-Gruppe anhand folgender Reaktionsgleichung erläutert :

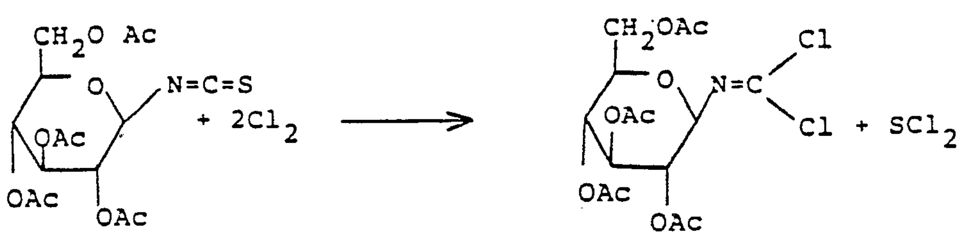

Ac = 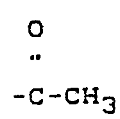

In α-Stellung durch eine Isothiocyanatgruppe substituierte cyclische Ether sind bekannt. Sie werden hergestellt, indem man in α-Stellung durch ein Halogenatom substituierte cyclische Ether mit bestimmten Schwermetallrhodaniden umsetzt ; gemäß der veröffentlichten japanischen Anmeldung

77/195123 wird als Schwermetallrhodanid Silberrhodanid verwendet, in Heterocycles 17, 87 (1982) wird die Verwendung von Bleirhodanid beschrieben. Nach dem in der DE-OS 33 41 018 beschriebenen Verfahren wird mit Alkalirhodaniden in Gegenwart von Phasentransferkatalysatoren gearbeitet.

Als Vertreter der für die Herstellung der erfindungsgemäßen cyclischen Ether der Formel I bevorzugt verwendeten Ausgangsverbindungen seien beispielsweise genannt :

2,3,4,6-Tetraacetyl-D-glucopyranosyl-1-isothiocyanat ;
2,3,4-Tribenzoyl-D-ribopyranosyl-1-isothiocyanat ;
2,3,5-Tribenzoyl-D-ribofuranosyl-1-isothiocyanat ;
2,2',3,3',4',6,6'-Heptaacetyl-D-lactosyl-1-isothiocyanat ;
2,2',3,3',4',6,6'-Heptaacetyl-D-cellobiosyl-1-isothiocyanat ;
3-Desoxy-3-chloro-2,4,6-triacetyl-D-glucosyl-1-isothiocyanat ;
2-Desoxy-2-(4-methyl)-phenylsulfonyloxy-3,4,6-triacetyl-D-glucosyl-1-isothiocyanat, aber auch 2,3,4-Triacetyl-6-desoxy-6-nitroglucopyranosyl-1-isothiocyanat und 2-Desoxy-3,4,6-tribenzoyl-D-glucopyranosyl-1-isothiocyanat,
2,3,4-Triacetyl-L-arabinopyranosyl-1-isothiocyanat und
2,3,4-Triacetyl-D-xylopyranosyl-1-isothiocyanat.

Das erfindungsgemäße Verfahren kann in Gegenwart inerter Lösungsmittel, d. h. Lösungsmitteln, die sich unter den Reaktionsbedingungen nicht verändern, durchgeführt werden.

Geeignete Lösungsmittel für das erfindungsgemäße Verfahren sind beispielsweise chlorierte aromatische Kohlenwasserstoffe wie 1,2-Dichlorbenzol, Trichlorbenzol ; nitrierte aromatische Kohlenwasserstoffe wie Nitrobenzol und halogenierte aliphatische Kohlenwasserstoffe wie Chloroform, Tetrachlorkohlenstoff, Tetrachlorethan und Pentachlorethan ; bevorzugt sind halogenierte aliphatische Kohlenwasserstoffe, insbesondere Chloroform und Tetrachlorkohlenstoff. Es können auch Gemische der Lösungsmittel eingesetzt werden.

Die Lösungsmittelmengen betragen im allgemeinen 1 bis 20, bevorzugt 2 bis 12, Gewichtsteile je Gewichtsteil cyclischen Ethers.

Das erfindungsgemäße Verfahren wird im allgemeinen bei Normaldruck durchgeführt. Es ist aber auch möglich, das erfindungsgemäße Verfahren bei einem Unter- oder Überdruck (beispielsweise im Druckbereich von 0,5 bis 10 bar) durchzuführen. Den Verlauf der Umsetzung kann man IR-spektroskopisch verfolgen (Verschwinden der NCS-Bande). Im allgemeinen ist die Umsetzung beendet, wenn die für die Umsetzung erforderlichen 2 Mol $Cl_2$ je Mol NCS-Gruppe eingeleitet worden sind.

Das erfindungsgemäße Verfahren kann beispielsweise wie folgt durchgeführt werden :

Der in α-Stellung durch eine Isothiocyanatgruppe substituierte cyclische Ether wird im Lösungsmittel vorgelegt. Nachdem die Mischung auf die gewünschte Reaktionstemperatur gebracht wurde, leitet man unter starkem Rühren Chlor bis zur Beendigung der Umsetzung ein. Anschließend destilliert man das Lösungsmittel und entstandenes Dichlorsulfan ab und isoliert den gewünschten in α-Stellung durch eine Isocyaniddichloridgruppe substituierten cyclischen Ether aus dem Rückstand nach an sich bekannten Methoden, z. B. durch Umkristallisieren.

Die in α-Stellung durch eine Isocyaniddichlorid-Gruppe substituierten cyclischen Ether der Formel I, insbesondere die acylierten Zucker mit glykosidisch gebundener Isocyaniddichlorid-Gruppe, sind wertvolle Zwischenprodukte für die Synthese biologisch aktiver Verbindungen. So eröffnet z. B. ihre Umsetzung mit entsprechenden Acylhydrazinen einen neuen wirtschaftlichen Weg zur Herstellung antiviral wirksamer 1,3,4-Oxadiazole (s. Acta Pol. Pharm. 1973, 30 (3), Seiten 255-260 ; vgl. CA 80, 83464e) ; die Umsetzung der Glykosylisocyanid-dichloride mit Natriumazid eröffnet einen neuen einfachen Weg zu antiviral wirksamen Glykosyltetrazolen (s. J. Med. Chem. 19 (2), 286 (1976) ; durch Umsetzung mit starken Säuren erhält man aus den acylierten Zuckern mit glykosidisch gebundener Isocyaniddichlorid-Gruppe in hohen Ausbeuten die entsprechenden acylierten Glykosylisocyanate. Diese acylierten Glykosylisocyanate sind bislang nur nach technisch unbrauchbaren Verfahren erhältlich (s. Coll. Czech. Chem. Comm. 29 (1969), Nr. 9, Seite 2060 und Z. Chemie 7 (1967), Nr. 5, Seite 183).

Diese Isocyanate sind von großem Interesse, weil aus ihnen durch Reaktion mit geeigneten Aminen antibakteriell wirksame Glykosylureide erhalten werden (s. DE-OS 25 09 260). Die Glykosylisocyanate sind weiterhin begehrte Ausgangsverbindungen für die Synthese biologisch aktiver heterocyclische N-Glykoside, wie sie z. B. in Heterocycles 17, 615 (1983) beschrieben sind.

## Beispiel 1

In eine Lösung von 192,5 g (0,5 Mol) 2,3,4,6-Tetraacetyl-β-D-glucosyl-1-isothiocyanat in 500 ml Chloroform wird bei 0 °C so lange Chlor eingeleitet, bis sich IR-spektroskopisch kein Isothiocyanat mehr nachweisen läßt. Man leitet noch einmal über die gleiche Zeit Chlor ein. Danach wird die Lösung im Vakuum bis zur Trockne eingeengt. Der Rückstand wird zur Kristallisation mit n-Hexan versetzt. Das Kristallisat wird in Chloroform aufgenommen und mit einer wäßrigen Lösung von $Na_2CO_3$ und $Na_2SO_3$ gewaschen. Nach dem Trocknen über $MgSO_4$ und Abfiltrieren wird das Lösungsmittel abgetrennt.

Es werden 198 g (≙ 92,5 % der Theorie) 2,3,4,6-Tetraacetyl-β-D-glucopyranosyl-1-isocyaniddichlorid erhalten ;

Fp. : 122-123 °C.

## Beispiel 2

In eine Lösung von 10,67 g (15,76 mmol) 2,2',3,3',4',6,6'-Heptaacetyl-β-D-cellobiosyl-1-isothiocyanat in 100 ml Chloroform wird 1 h lang Chlor bei 0 °C eingeleitet. Die Lösung wird zur Trockne eingeengt. Der Rückstand wird mit 100 ml n-Hexan verrührt. Das Kristallisat wird abgesaugt und getrocknet.

Es werden 9,2 g (≙ 81,5 % der Theorie) 2,2',3,3',4',6,6'-Heptaacetyl-β-D-cellobiosyl-1-isocyaniddichlorid erhalten ;

Fp. : 220 °C (Z).

## Beispiel 3

9,2 g (18,3 mmol) 2,3,5-Tribenzoyl-β-D-ribofuranosyl-1-isothiocyanat werden in 80 ml Chloroform gelöst und bei 0 °C 1 h chloriert. Man trennt Lösungsmittel und entstandenes Dichlorsulfan ab. Der Rückstand wird mit 100 ml n-Hexan verrührt, wobei der kristallisiert. Das Kristallisat wird abgesaugt und getrocknet.

Es werden 8,9 g (= 89,7 % der Theorie) 2,3,5-Tribenzoyl-β-D-ribofuranosyl-1-isocyaniddichlorid erhalten ;

Fp. : 90-93 °C.

## Beispiel 4

Eine Lösung von 5 g (10 mmol) 2-Desoxy-2-p-toluol-sulfonyloxy-3,4,6-triacetyl-β-D-glucopyranosyl-1-isothiocyanat in 20 ml Chloroform wird bei 0 °C chloriert. Nach Abtrennung des Lösungsmittels und des Dichlorsulfans wird der Rückstand in wenig Essigester gelöst und wieder mit Hexan ausgefällt. Das ausfallende Öl liefert nach dem Abdekantieren des Lösungsmittels und Entfernen der Lösungsmittelreste im Vakuum einen hellgelben Schaum, der sich durch Verrühren mit Hexan in ein amorphes Pulver überführen läßt.

Es werden 4,5 g (= 85 % der Theorie) 2-Desoxy-2-p-toluolsulfonyloxy-3,4,6-triacetyl-β-D-glucopyranosyl-1-isocyaniddichlorid in Form eines amorphen Pulvers erhalten.

IR : 1 750 $cm^{-1}$ (Ester) ; 1 660 $cm^{-1}$

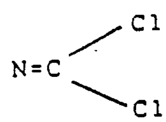

## Beispiel 5

19,25 g (50 mmol) 2,3,4,6-Tetraacetyl-β-D-glucopyranosyl-1-isothiocyanat werden in 100 ml Tetrachlorkohlenstoff gelöst und, wie in Beispiel 1 beschrieben, chloriert. Nach dem Einengen der Reaktionslösung und dem Verrühren des Rückstandes mit Hexan werden 19 g (= 98,6 % der Theorie) 2,3,4,6-Tetraacetyl-β-D-glucopyranosyl-isocyaniddichlorid erhalten ;

Fp. : 122-123 °C.

Führt man die Reaktion statt in Tetrachlorkohlenstoff in 100 ml 1,2-Dichlorbenzol durch, so beträgt die Ausbeute an 2,3,4,6-Tetraacetyl-β-D-glucopyranosyl-isocyaniddichlorid 92 % der Theorie.

## Beispiel 6

Die Lösung von 10,4 g (32,9 mmol) 2,3,4-Tetraacetyl-β-L-arabinopyranosyl-1-isothiocyanat in 100 ml Chloroform wird, wie in Beispiel 1 beschrieben, chloriert. Nachdem sich das Reaktionsgemisch auf Raumtemperatur erwärmt hat, entfernt man Lösungsmittel und Dichlorsulfan im Vakuum. Der Rückstand wird in Diisopropylether aufgenommen, die Lösung mit Aktivkohle verrührt und filtriert. Aus dem Filtrat wird der Diisopropylether im Vakuum entfernt. Es werden 9,2 g (78,4 % der Theorie) 2,3,4-Triacetyl-β-L-arabinopyranosyl-1-isocyaniddichlorid in Form eines hochviskosen Öls erhalten.

## Beispiel 7

Die Lösung von 2,5 g (7,9 mmol) 2,3,4-Triacetyl-β-D-xylopyranosyl-1-isothiocyanat in 50 ml Chloroform wird, wie in Beispiel 1 beschrieben, chloriert. Nach dem Entfernen des Lösungsmittels und des Dichlorsulfans im Vakuum wird der Rückstand zur Kristallisation mit n-Hexan versetzt. Man erhält 1,5 g (= 53 % der Theorie) 2,3,4-Triacetyl-β-D-xylopyranosyl-1-isocyaniddichlorid ;

Fp. : 117 °C.

## Beispiel 8 (Anwendungsbeispiel)

Die Lösung von 85,6 g (0,2 Mol) 2,3,4,6-Tetraacetyl-β-D-glucopyranosyl-1-isocyaniddichlorid in 400 ml

Chlorbenzol wird mit 19,2 g (0,2 Mol) Methansulfonsäure versetzt. Das Gemisch wird auf 100 °C erhitzt. Nach 40 Minuten ist die Reaktion beendet. Lösungsmittel und entstandenes Methansulfonylchlorid werden im Vakuum abdestilliert. Der Rückstand wird aus Xylol umkristallisiert.

Es werden 54,6 g (= 73 % der Theorie) 2,3,4,6-Tetraacetyl-β-D-glucopyranosyl-1-isocyanat erhalten ; Fp. : 113-115 °C.

Beispiel 9 (Anwendungsbeispiel)

Zu einer auf 0 °C gekühlten Lösung von 42,8 g (0,1 Mol) 2,3,4,6-Tetra-O-acetyl-β-D-glucopyranosyl-1-isocyaniddichlorid und 101 g (1 Mol) Triethylamin in 200 ml Dimethylformamid tropft man innerhalb 10 Minuten eine Lösung von 20,5 g (0,1 Mol) 3,4-Dichlorbenzoylhydrazin in 70 ml Dimethylformamid. Man rührt 15 Minuten bei Zimmertemperatur, saugt ab und engt das Filtrat ein. Der Rückstand wird in Chloroform aufgenommen und mit Wasser gewaschen. Die Chloroformphase wird getrocknet und eingeengt. Das Produkt wird aus Ethanol umkristallisiert.

Man erhält 28,5 g (= 50,7 % der Theorie) 2-(3,4-Dichlorphenyl)-5-(2,3,4,6-tetra-O-acetyl-β-D-glucopyranosyl)-amino-1,3,4-oxadiazol ;

Fp. : 180-181 °C.

Beispiel 10 (Anwendungsbeispiel)

Zu einer Lösung von 20 g (0,0467 Mol) 2,3,4,6-Tetra-O-acetyl-β-D-glucopyranosyl-1-isocyaniddichlorid in 150 ml Dimethylformamid gibt man 3,035 g (0,0467 Mol) Natriumazid und rührt 30 Minuten. Man saugt ab, engt das Filtrat ein und kristallisiert den erhaltenen Sirup aus Ethanol um. Man erhält 10 g (50 % der Theorie) 1-(2,3,4,6-Tetra-O-acetyl-β-D-glucopyranosyl)-5-chlor-tetrazol ;

Fp. : 172 °C.

**Patentansprüche**

1. In α-Stellung durch eine Isocyaniddichlorid-Gruppe substituierte cyclische Ether der Formel

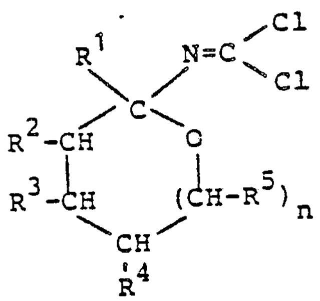

(I)

in der

$R^1$ eine Acyloxymethylgruppe oder Wasserstoff bedeutet,

n 0 oder 1 ist und

$R^2$, $R^3$, $R^4$ und $R^5$ unabhängig voneinander für Wasserstoff, Halogen, eine Nitro-, Acyloxy- oder Acyloxymethyl-Gruppe oder den Rest eines cyclischen Ethers der Formel

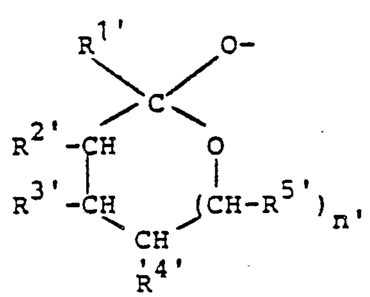

(II)

stehen, in der n', $R^{1'}$, $R^{2'}$, $R^{3'}$, $R^{4'}$ und $R^{5'}$ unabhängig voneinander und unabhängig von den ihnen entsprechenden Werten n, $R^1$, $R^2$, $R^3$, $R^4$ und $R^5$ in Formel I, die für n, $R^1$, $R^2$, $R^3$, $R^4$, $R^5$ unter Formel I angegebene Bedeutung haben, mit der Maßgabe, daß mindestens einer der Reste $R^1$, $R^2$, $R^3$, $R^4$ und $R^5$ und mindestens einer der Reste $R^{1'}$, $R^{2'}$, $R^{3'}$, $R^{4'}$ und $R^{5'}$ eine Acyloxymethyl- oder Acyloxy-Gruppe ist

und daß höchstens einer der Reste $R^2$, $R^3$, $R^4$ oder $R^5$ und höchstens einer der Reste $R^{2'}$, $R^{3'}$, $R^{4'}$ oder $R^{5'}$ für den Rest eines cyclischen Ethers der Formel II steht.

2. Cyclische Ether gemäß Anspruch 1, dadurch gekennzeichnet, daß in Formel I $R^1$ Wasserstoff und n 0 oder 1 ist und mindestens zwei der Reste $R^1$, $R^2$, $R^3$, $R^4$ und $R^5$ und mindestens zwei der Reste $R^{1'}$, $R^{2'}$, $R^{3'}$, $R^{4'}$ und $R^{5'}$ eine Acyloxymethyl- oder Acyloxy-Gruppe sind.

3. Cyclische Ether gemäß Anspruch 1, dadurch gekennzeichnet, daß in Formel I
$R^1$ Wasserstoff und
n 0 oder 1 ist und
$R^2$, $R^3$, $R^4$ und $R^5$ für eine Acyloxy- oder Acyloxymethyl-Gruppe stehen oder einer der Reste $R^2$, $R^3$, $R^4$ oder $R^5$ der Rest eines cyclischen Ethers der Formel II ist, und die verbleibenden Reste $R^2$, $R^3$, $R^4$, $R^5$ für eine Acyloxy- oder eine Acyloxymethyl-Gruppe stehen, wobei in Formel II $R^{1'}$ für Wasserstoff oder eine Acyloxymethylgruppe, n' für 0 oder 1 und $R^{2'}$, $R^{3'}$, $R^{4'}$ und $R^{5'}$ für eine Acyloxy- oder eine Acyloxymethyl-Gruppe stehen.

4. Cyclische Ether gemäß Ansprüchen 1 bis 3, dadurch gekennzeichnet, daß sich die Acylreste in den Acyloxy- oder Acyloxymethyl-Gruppen von $C_1$-$C_8$-Alkancarbonsäureen oder aromatischen Carbonsäuren ableiten.

5. Verfahren zur Herstellung von in α-Stellung durch eine Isocyaniddichloridgruppe substituierten cyclischen Ethern der Formel

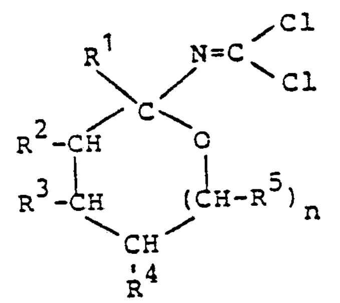

(I)

in der
$R^1$ eine Acyloxymethylgruppe oder Wasserstoff bedeutet,
n 0 oder 1 ist und
$R^2$, $R^3$, $R^4$ und $R^5$ unabhängig voneinander für Wasserstoff, Halogen, eine Nitro-, Acyloxy- oder Acyloxymethyl-Gruppe oder den Rest eines cyclischen Ethers der Formel

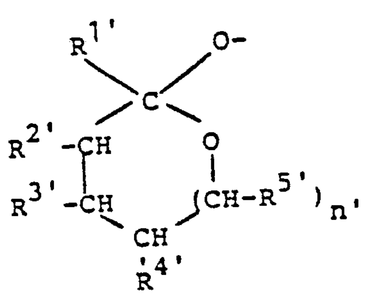

(II)

stehen, in der n', $R^{1'}$, $R^{2'}$, $R^{3'}$, $R^{4'}$ und $R^{5'}$ unabhängig voneinander und unabhängig von den ihnen entsprechenden Werten n, $R^1$, $R^2$, $R^3$, $R^4$ und $R^5$ in Formel I, die für n, $R^1$, $R^2$, $R^3$, $R^4$, $R^5$ unter Formel I angegebene Bedeutung haben, mit der Maßgabe, daß mindestens einer der Reste $R^1$, $R^2$, $R^3$, $R^4$ und $R^5$ und mindestens einer der Reste $R^{1'}$, $R^{2'}$, $R^{3'}$, $R^{4'}$ und $R^{5'}$ eine Acyloxymethyl- oder Acyloxy-Gruppe ist und daß höchstens einer der Reste $R^2$, $R^3$, $R^4$ oder $R^5$ und höchstens einer der Reste $R^{2'}$, $R^{3'}$, $R^{4'}$ oder $R^{5'}$ für den Rest eines cyclischen Ethers der Formel II steht,
dadurch gekennzeichnet, daß man in α-Stellung durch eine Isothiocyanat-Gruppe substituierte cyclische Ether der Formel

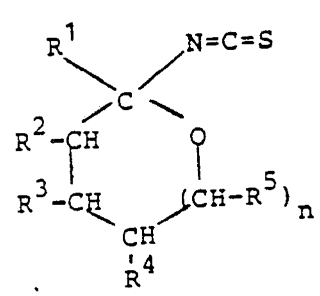

in der n, $R^1$, $R^2$, $R^3$, $R^4$ und $R^5$ die vorstehend angegebene Bedeutung haben, in einem inerten Lösungsmittel bei Temperaturen von —40 °C bis + 80 °C mit mindestens 2 Mol Chlor je Mol Isothiocyanat-Gruppe umsetzt.

6. Verfahren gemäß Anspruch 5, dadurch gekennzeichnet, daß man je Mol Isothiocyanat-Gruppe 2 bis 6 Mol Chlor einsetzt.

7. Verfahren gemäß Anspruch 5 oder 6, dadurch gekennzeichnet, daß man die Umsetzung bei —20 °C bis + 60 °C vornimmt.

8. Verfahren gemäß Ansprüchen 5 bis 7, dadurch gekennzeichnet, daß man 1 bis 20 Gewichtsteile Lösungsmittel je Gewichtsteil cyclischen Ethers verwendet.

**Claims**

1. Cyclic ethers, substituted in the α-position by an isocyanide-dichloride group, of the formula

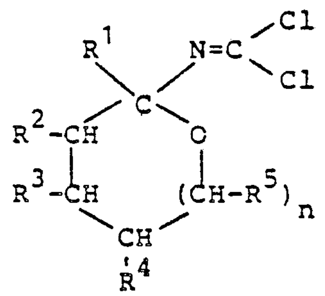

(I)

in which

$R^1$ denotes an acyloxymethyl group or hydrogen,

n is 0 or 1 and

$R^2$, $R^3$, $R^4$ and $R^5$ independently of one another represent hydrogen, halogen, a nitro, acyloxy or acyloxymethyl group or the radical of a cyclic ether of the formula

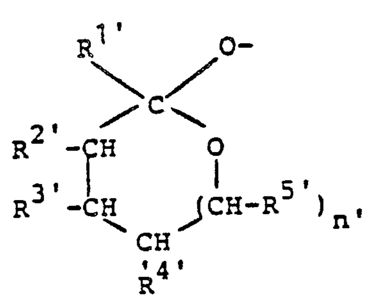

(II)

in which n', $R^{1'}$, $R^{2'}$, $R^{3'}$, $R^{4'}$ and $R^{5'}$, independently of one another and independently of the values n, $R^1$, $R^2$, $R^3$, $R^4$ and $R^5$ in formula I which correspond to them, have the meaning indicated for n, $R^1$, $R^2$, $R^3$, $R^4$ and $R^5$ under formula I, subject to the proviso that at least one of the radicals $R^1$, $R^2$, $R^3$, $R^4$ and $R^5$ and at least one of the radicals $R^{1'}$, $R^{2'}$, $R^{3'}$, $R^{4'}$ and $R^{5'}$ is an acyloxymethyl or acyloxy group and that not more than one of the radicals $R^2$, $R^3$, $R^4$ or $R^5$ and not more than one of the radicals $R^{2'}$, $R^{3'}$, $R^{4'}$ or $R^{5'}$ represents the radical of a cyclic ether of the formula II.

2. Cyclic ethers according to Claim 1, characterised in that, in formula I, $R^1$ is hydrogen and n is 0 or 1 and at least two of the radicals $R^1$, $R^2$, $R^3$, $R^4$ and $R^5$ and at least two of the radicals $R^{1'}$, $R^{2'}$, $R^{3'}$, $R^{4'}$ and $R^{5'}$ are an acyloxymethyl or acyloxy group.

3. Cyclic ethers according to Claim 1, characterised in that, in formula I,

$R^1$ is hydrogen and

n is 0 or 1 and

$R^2$, $R^3$, $R^4$ and $R^5$ represent an acyloxy or acyloxymethyl group, or one of the radicals $R^2$, $R^3$, $R^4$ or $R^5$ is the radical of a cyclic ether of the formula II, and the remaining radicals $R^2$, $R^3$, $R^4$ and $R^5$ represent an acyloxy group or an acyloxymethyl group, and, in formula II, $R^{1'}$ represents hydrogen or an acyloxymethyl group, n' represents 0 or 1 and $R^{2'}$, $R^{3'}$, $R^{4'}$ and $R^{5'}$ represent an acyloxy group or an acyloxymethyl group.

4. Cyclic ethers according to Claims 1 to 3, characterised in that the acyl radicals in the acyloxy or acyloxymethyl groups are derived from $C_1$-$C_8$-alkanecarboxylic acids or aromatic carboxylic acids.

5. Process for the preparation of cyclic ethers, substituted in the α-position by an isocyanide-dichloride group, of the formula

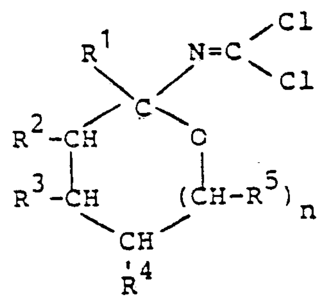

(I)

in which

$R^1$ denotes an acyloxymethyl group or hydrogen,

n is 0 or 1 and

$R^2$, $R^3$, $R^4$ and $R^5$ independently of one another represent hydrogen, halogen, a nitro, acyloxy or acyloxymethyl group or the radical of a cyclic ether of the formula

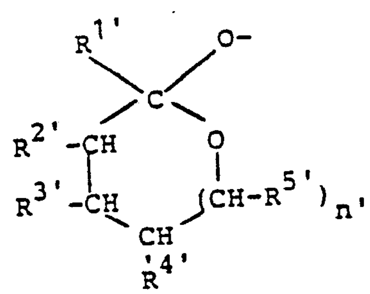

(II)

in which n', $R^{1'}$, $R^{2'}$, $R^{3'}$, $R^{4'}$ and $R^{5'}$ independently of one another and independently of the values n, $R^1$, $R^2$, $R^3$, $R^4$ and $R^5$ in formula I which correspond to them, have the meaning indicated for n, $R^1$, $R^2$, $R^3$, $R^4$ and $R^5$ under formula I, subject to the proviso that at least one of the radicals $R^1$, $R^2$, $R^3$, $R^4$ and $R^5$ and at least one of the radicals $R^{1'}$, $R^{2'}$, $R^{3'}$, $R^{4'}$ and $R^{5'}$ is an acyloxymethyl or acyloxy group, and that not more than one of the radicals $R^2$, $R^3$, $R^4$ or $R^5$ and not more than one of the radicals $R^{2'}$, $R^{3'}$, $R^{4'}$ or $R^{5'}$ represents the radical of a cyclic ether of the formula II,

characterised in that cyclic ethers, substituted in the α-position by an isothiocyanate group, of the formula

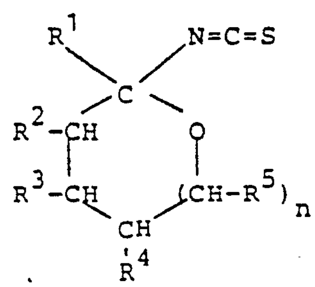

in which n, $R^1$, $R^2$, $R^3$, $R^4$ and $R^5$ have the meaning indicated above, are reacted, in an inert solvent at temperatures of —40 °C to + 80 °C, with at least 2 moles of chlorine per mole of isothiocyanate group.

6. Process according to Claim 5, characterised in that 2 to 6 moles of chlorine are employed per mole of isothiocyanate group.

7. Process according to Claim 5 or 6, characterised in that the reaction is carried out at — 20 °C to + 60 °C.

8. Process according to claims 5 to 7, characterised in that 1 to 20 parts by weight of solvent are used per part by weight of cyclic ether.

**Revendications**

1. Ethers cycliques substitués en position α par un groupe bichlorure d'isocyanure et répondant à la formule

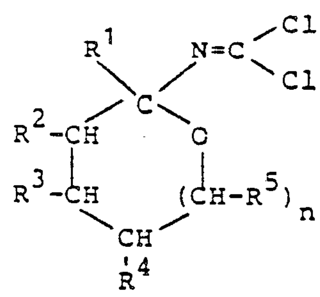

(I)

dans laquelle

$R^1$ représente un groupe acyloxyméthyle ou de l'hydrogène,

n représente 0 ou 1 et

$R^2$, $R^3$, $R^4$ et $R^5$ représentent, indépendamment les uns des autres, de l'hydrogène, un halogène, un groupe nitro, acyloxy ou acyloxyméthyle, ou bien le résidu d'un éther cyclique répondant à la formule

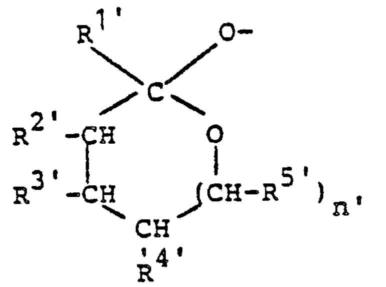

(II)

dans laquelle n', $R^{1'}$, $R^{2'}$, $R^{3'}$, $R^{4'}$ et $R^{5'}$ ont, indépendamment les uns des autres et indépendamment de leurs valeurs correspondantes, n, $R^1$, $R^2$, $R^3$, $R^4$ et $R^5$ dans la formule I, les significations mentionnées pour n, $R^1$, $R^2$, $R^3$, $R^4$, et $R^5$ à propos de la formule I avec pour condition qu'au moins l'un des résidus $R^1$, $R^2$, $R^3$, $R^4$ et $R^5$ et au moins l'un des résidus $R^{1'}$, $R^{2'}$, $R^{3'}$, $R^{4'}$ et $R^{5'}$ représente un groupe acyloxyméthyle ou acyloxy et qu'au plus un des résidus $R^2$, $R^3$, $R^4$ ou $R^5$ et au plus un des résidus $R^{2'}$, $R^{3'}$, $R^{4'}$ ou $R^{5'}$ représente le résidu d'un éther cyclique de la formule II.

2. Ethers cycliques selon la revendication 1, caractérisés en ce que, dans la formule I $R^1$ représente de l'hydrogène et n 0 ou 1 et qu'au moins deux des résidus $R^1$, $R^2$, $R^3$, $R^4$ et $R^5$ et au moins deux des résidus $R^{1'}$, $R^{2'}$, $R^{3'}$, $R^{4'}$ et $R^{5'}$ représentent un groupe acyloxyméthyle ou acyloxy.

3. Ethers cycliques selon la formule 1, caractérisés en ce que, dans la formule I

$R^1$ représente de l'hydrogène et

n 0 ou 1 et que

$R^2$, $R^3$, $R^4$ et $R^5$ représentent un groupe acyloxy ou acyloxyméthyle ou l'un des résidus $R^2$, $R^3$, $R^4$ ou $R^5$ représente le résidu d'un éther cyclique de la formule II et que les résidus restants $R^2$, $R^3$, $R^4$ et $R^5$ représentent un groupe acyloxy ou acyloxyméthyle, tandis que dans la formule II, $R^{1'}$ représente de l'hydrogène ou un groupe acyloxyméthyle, n' représente 0 ou 1 et $R^{2'}$, $R^{3'}$, $R^{4'}$ et $R^{5'}$ représentent un groupe acyloxy ou acyloxyméthyle.

4. Ethers cycliques selon les revendications 1 à 3, caractérisés en ce que les résidus acylés dans les groupes acyloxy ou acyloxyméthyle dérivent des acides alcanecarboxyliques en $C_1$-$C_8$ ou des acides carboxyliques aromatiques.

5. Procédé de production des éthers cycliques substitués en position α par un groupe bichlorure d'isocyanure et répondant à la formule

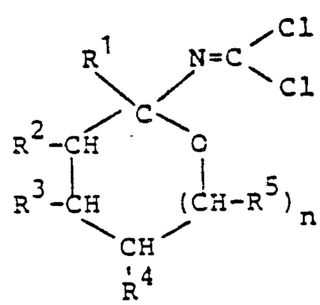

(I)

dans laquelle

$R^1$ représente de l'hydrogène ou un groupe acyloxyméthyle,

n représente 0 ou 1 et

$R^2$, $R^3$, $R^4$ et $R^5$ représentent, indépendamment les uns des autres, de l'hydrogène, un halogène, un groupe nitro, acyloxy ou acyloxyméthyle, ou encore le résidu d'un éther cyclique de la formule

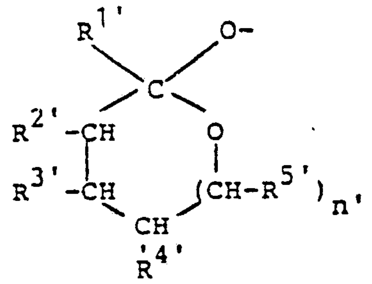

(II)

dans laquelle n', $R^{1'}$, $R^{2'}$, $R^{3'}$, $R^{4'}$ et $R^{5'}$ ont, indépendamment les uns des autres et indépendamment de leurs valeurs correspondantes n, $R^1$, $R^2$, $R^3$, $R^4$ et $R^5$ dans la formule 1, la signification mentionnée pour n, $R^1$, $R^2$, $R^3$, $R^4$ et $R^5$ à propos de la formule I avec pour condition qu'au moins un des résidus $R^1$, $R^2$, $R^3$, $R^4$ et $R^5$ et au moins un des résidus $R^{1'}$, $R^{2'}$, $R^{3'}$, $R^{4'}$ et $R^{5'}$ représente un groupe acyloxyméthyle ou acyloxy et qu'au plus l'un des résidus $R^2$, $R^3$, $R^4$ ou $R^5$ et au plus l'un des résidus $R^{2'}$, $R^{3'}$, $R^{4'}$ ou $R^{5'}$ représente le résidu d'un éther cyclique de la formule II, caractérisé en ce que l'on fait réagir des éthers cycliques substitués en position α par un groupe isothiocyanate répondant à la formule

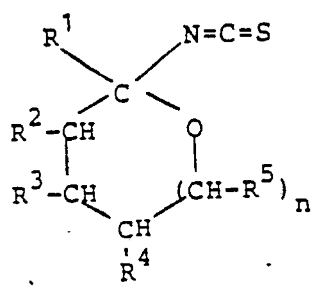

dans laquelle n, $R^1$, $R^2$, $R^3$, $R^4$ et $R^5$ ont les significations mentionnées ci-dessus, dans un solvant inerte à des températures de $-40\,°C$ à $+80\,°C$ avec au moins 2 moles de chlore par mole de groupe isothiocyanate.

6. Procédé selon la revendication 5, caractérisé en ce que l'on utilise de 2 à 6 moles de chlore par mole de groupe isothiocyanate.

7. Procédé selon la revendication 5 ou 6, caractérisé en ce que l'on effectue la réaction entre $-20\,°C$ et $+60\,°C$.

8. Procédé selon les revendications 5 à 7, caractérisé en ce que l'on utilise de 1 à 20 parties en poids de solvant par partie en poids d'éther cyclique.